# EUROPEAN PATENT APPLICATION

(11) **EP 4 603 516 A1**
(43) Date of publication of application: **20.08.2025**
(21) Application number: 25151411.3
(22) Date of filing: 13.01.2025
(51) Int. Cl.: C08F 2/50, C07C 49/00, C08F 220/20, C09D 11/38, G03F 7/004, C07C 69/76, C08F 2/48, C09D 11/101, C09D 11/03

(54) **PHOTOINITIATOR AND CURABLE COMPOSITION COMPRISING THE SAME**

(30) Priority: 18.01.2024 EP 24152630
(71) Applicant: Covestro (Taiwan) Ltd, Taipei 11049 (TW); National Central University, Taoyuan City 320317 (TW)
(72) Inventor: WEN, Wen Hsien, 320317 Taoyuan City (TW); KE, Yao Sheng, 320317 Taoyuan City (TW); JANSEN, Johan Franz Gradus Antonius, 6165 AP Geleen (NL); CHEN, Ming-Chou, 320317 Taoyuan City (TW); PAN, Hao-Wei, 320317 Taoyuan City (TW); LIN, Jui-Han, 320317 Taoyuan City (TW)
(74) Representative: Levpat

(57) **Abstract**

The invention provides a photoinitiator which is prepared by ring-opening reaction of epoxidized vegetable oil with one or more Norrish II photoinitiators having a carboxylic acid group, hydroxyl group, mercaptan group and/or amine group, and one or more acrylic compounds. The invention also provides a photocurable composition comprising the photoinitiator. The photoinitiator of the present invention has the advantages of low migration, non-toxicity, low volatility, thermal stability and easy dissolution in organic solvents.

## Description

### BACKGROUND

### Field of the Disclosure

The present invention relates to a photoinitiator and a photocurable composition including the same.

### Description of the Related Art

Since the 1990s, UV curing has been substantially developed due to the advent of photoinitiators such as isopropylthioxanthone (ITX), 2,4,6-trimethylbenzoyldiphenyl phosphine oxide (TPO) and phenyl bis(2,4,6-trimethylbenzoyl)phosphine oxide (also known as Photoinitiator 819). UV curing coatings are those that, when irradiated with UV light, cause the liquid resin monomers in the coating composition to polymerize and transform into a solid film at room temperature. Compared with traditional thermal curing, UV curing coatings have the advantages including high curing speed, room temperature curing, energy saving, floor space saving, no environment pollution and higher product performance, and being suitable for high-speed automatic production.

Commercially available photoinitiators mainly include benzophenone (BP), ITX and TPO, but these photoinitiators are often odorous, and may easily migrate from the printed matter to its surface, causing harm to human health. One way to overcome the above problems is to modify the photoinitiator, e.g., by increasing the molecular weight to reduce migration.

In addition, epoxidized vegetable oil is an extensive biobased raw material which is low in cost and has certain chemical reactivity, and can be used as a structural basis for the preparation of polymer materials. For example, epoxidized soybean oil (ESBO) is an epoxidized vegetable oil product prepared by epoxidizing soybean oil.

Photoinitiators have been introduced into epoxidized soybean oil (ESBO) for modification to increase the molecular weight. For example, China Patent Publication No. CN101705044B discloses a soybean oil-methylpropiophenone photoinitiator, in which 2-hydroxy-2-methylpropiophenone reacts with epoxidized soybean oil (ESBO) to form the soybean oil photoinitiator. For another example, the journal article published by Jie Yin et al. in 2012 (Aifang Luo, Xuesong Jiang, Jie Yin, "Thioxanthone-containing renewable vegetable oil as photoinitiators," Polymer. 2012, 53, 2183-2189) discloses a soybean oil-thioxanthone photoinitiator, in which soybean oil reacts with 2-acetic thioxanthone (ATX) as a photoinitiator and 4-dimethylaminobenzoic acid (DBA) as an amine synergist to prepare the soybean oil-thioxanthone photoinitiator (ESBO-ATX-DBA).

However, the reactivity of these photoinitiators is still insufficient, and in order to achieve a desired curing speed, a considerable amount of these photoinitiators is usually needed. In addition, an excessive use of the photoinitiator will lead to the failure to form cured solids and an increase in viscosity as well as an increase in migration.

Therefore, there is still a need in industry for a modified photoinitiator with low migration and high reactivity, to facilitate subsequent industrial applications.

### SUMMARY

An objective of the present invention is to provide a photoinitiator, which is prepared by a ring-opening reaction of epoxidized vegetable oil with one or more Norrish Type II photoinitiators having carboxylic acid group, hydroxyl group, thiol group and/or amino group and one or more acrylic compounds.

Another objective of the present invention is to provide a photoinitiator, which is prepared by a ring-opening reaction of epoxidized vegetable oil with one or more acrylic compounds and one or more compounds of formula (II), wherein 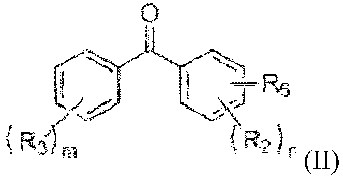
R₂ and R₃ are independently selected from C₁₋₆ alkyl, C₁₋₆ alkoxy, hydroxyl, halogen, -COO-C₁₋₆ alkyl, C₆₋₁₀ aryl, or -NR₄R₄; or R₂ and R₃ together form -S-, -O-, -CO- or -C=C-;
m is 0, 1, 2, 3 or 4;
n is 0, 1, 2 or 3;
R₆ is selected from -COOH, -OH, -SH, -NR₄R₄, -CORsOH or -OR₅-COOH;
R₄ is independently selected from H or C₁₋₆ alkyl; and
R₅ is selected from C₁₋₂₀ alkylene or C₂₋₂₀ alkenylene unsubstituted or substituted with one or more hydroxyl, halogen, C₁₋₆ alkoxy, or acyloxy groups.

In case m is higher than 1, the R₃ groups can be part of a ring structure. In case n is higher than 1, the R₂ groups can be part of a ring structure. Preferably n and m are 0.

Another objective of the present invention is to provide a photoinitiator according to idealized formula (III): 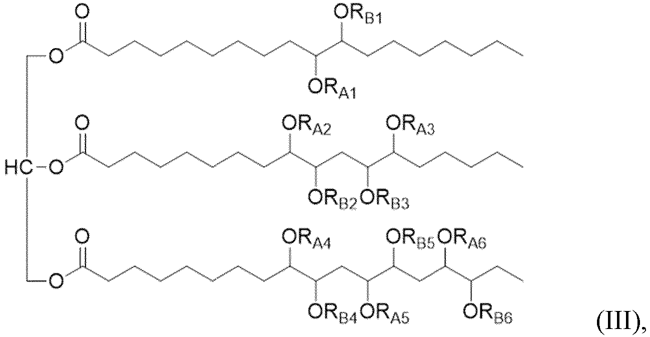 wherein R_{A1}, R_{B1}, R_{A2}, R_{B2}, R_{A3}, R_{B3}, R_{A4}, R_{B4}, R_{A5}, R_{B5}, R_{A6} and R_{B6} are independently selected from 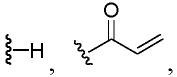 and 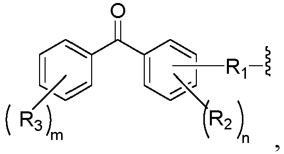 with the proviso that
(1) The total amount of 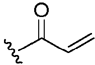 and 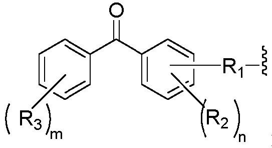 is from 4 to 6;
(2) The total amount of 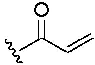 is from 1 to 5;
(3) The total amount of 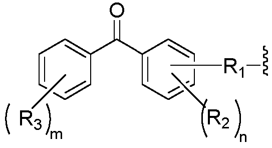 is from 1 to 5;
(4) For each i, wherein i is from 1 to 6: if R_{Ai} is 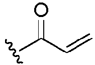 or 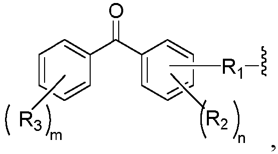 then the corresponding R_{Bi} is 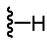 ; and
(5) For each i, wherein i is from 1 to 6: if R_{Bi} is 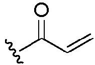 or 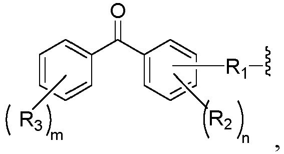 then the corresponding R_{Ai} is

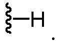

Another objective of the present invention is to provide a photocurable composition, including an ethylenically unsaturated curable compound and a photoinitiator as described above.

The present invention can effectively solve the above problems by preparing the photoinitiator from the epoxidized vegetable oil, and develop products with more diversified applications. According to the present invention, through the reaction of the epoxidized vegetable oil with a Norrish Type II photoinitiator, such as benzophenone, and an acrylic compound such as acrylic acid, a photoinitiator having higher molecular weight can be prepared. Compared with the traditional photoinitiators, the photoinitiator of the present invention has the characteristics of high reactivity, low migration, no toxicity and low volatility, has biobased units, and has the advantage of high efficiency.

### DETAILED DESCRIPTION

The present disclosure is described with reference to the following embodiments. Aside from the following embodiments, the present disclosure may be performed according to another method without departing from the spirit of the present disclosure.

For ease of understanding of the disclosure of this specification, a plurality of terms are defined as follows. The terminology used herein is for the purpose of describing particular embodiments only and is not intended to be limiting of the invention. The singular forms are intended to include the plural forms as well, unless the context clearly indicates otherwise. It will be further understood that the terms "include," "have" or "comprise" when used in this specification, specify the presence of stated features, numbers, steps, components, or combinations thereof, but do not preclude the presence or addition of one or more other features, numbers, steps, components, or combinations thereof.

All numerical values expressing the contents, proportions, and physical characteristics, etc. used in the specification and claims are to be construed as being modified by the term "about." Herein, the term "about" refers to an acceptable error of a specific value determined by those skilled in the art, depending in part on how the value is measured or determined.

The present invention provides a photoinitiator, which is prepared by a ring-opening reaction of epoxidized vegetable oil with one or more Norrish Type II photoinitiators having carboxylic acid group, hydroxyl group, thiol group and/or amino group and one or more acrylic compounds.

As used herein, the epoxidized vegetable oil may be any product obtained by epoxidizing unsaturated vegetable oil, which may include, but not limited to, epoxidized soybean oil, cottonseed oil, rapeseed oil, corn oil, peanut oil, sunflower seed oil and/or safflower seed oil. In an embodiment of the present invention, the epoxidized vegetable oil is epoxidized soybean oil.

As used herein, the Norrish Type II photoinitiators are those known in the art, preferably aromatic ketones. Examples of suitable aromatic ketones include, but not limited to, benzophenone and its derivatives.

The term "alkyl" as used herein refers to straight chain and branched saturated hydrocarbon groups having from 1 to 20 carbon atoms or, 1 to 12, 1 to 8, 1 to 6, or 1 to 4 carbon atoms. Alkyl groups further include cycloalkyl groups. Examples of straight chain alkyl groups include those with from 1 to 8 carbon atoms such as methyl, ethyl, n-propyl, n-butyl, n-pentyl, n-hexyl, n-heptyl, and n-octyl groups. Examples of branched alkyl groups include, but are not limited to, isopropyl, iso-butyl, sec-butyl, tert-butyl, neopentyl, isopentyl, and 2,2-dimethylpropyl groups. Additionally, the alkyl group may be optionally substituted.

The term "alkenyl" as used herein refers to straight chain and branched groups having from 1 to 20 carbon atoms or, 1 to 12, 1 to 8, 1 to 6, or 1 to 4 carbon atoms, and at least one C=C bonding. Additionally, the alkenyl group may be optionally substituted.

The terms "alkylene" and "alkenylene," as used herein refer to divalent radicals which are derived from the respective monovalent radicals by further abstraction of a hydrogen. For example, alkylene is derived from alkyl.

The term "alkoxy" refers to the group -O-alkyl. Examples include methoxy, ethoxy, propoxy, isopropoxy, cyclopropyloxy, cyclohexyloxy and the like.

The term "acyloxy" as used herein refers to a R(C=O)O- radical wherein "R" can be alkyl, alkenyl, alkynyl, heteroalkyl, heteroalkenyl, heteroalkynyl, aryl, cyclohexyl, heteroaryl or heterocycloalkyl. The acyloxy group is attached to the parent molecular structure through the oxygen functionality.

The term "alkynyl" refers to a straight or branched hydrocarbon chain radical group consisting solely of carbon and hydrogen atoms, containing at least one triple bond, having from two to ten carbon atoms (i.e., C₂-C₁₀ alkynyl). The alkynyl is attached to the parent molecular structure by a single bond, for example, ethynyl, propynyl, butynyl, pentynyl, hexynyl, and the like.

As used herein, "heteroalkyl," "heteroalkenyl" and "heteroalkynyl" include alkyl, alkenyl and alkynyl radicals, respectively, which have one or more skeletal chain atoms selected from an atom other than carbon, e.g., oxygen, nitrogen, sulfur, phosphorus or combinations thereof.

As used herein, "aryl" or "aromatic" groups are cyclic aromatic hydrocarbons that do not contain heteroatoms. Aryl groups include monocyclic, bicyclic and polycyclic ring systems. Thus, aryl groups include, but are not limited to, phenyl, azulenyl, heptalenyl, biphenylenyl, indacenyl, fluorenyl, phenanthrenyl, triphenylenyl, pyrenyl, naphthacenyl, chrysenyl, biphenyl, anthracenyl, indenyl, indanyl, pentalenyl, and naphthyl groups. In some embodiments, aryl groups contain 6-14 carbons, and in others from 6 to 12 or even 6-10 carbon atoms in the ring portions of the groups. The phrase "aryl groups" includes groups containing fused rings, such as fused aromatic-aliphatic ring systems (e.g., indanyl, tetrahydronaphthyl, and the like). Aryl groups may be substituted or unsubstituted.

As used herein, "heteroaryl" or, alternatively, "heteroaromatic" refers to a radical of a 5-18 membered monocyclic or polycyclic (e.g., bicyclic or tricyclic) aromatic ring system (e.g., having 6, 10 or 14 π (pi) electrons shared in a cyclic array) having ring carbon atoms and 1-6 ring heteroatoms provided in the aromatic ring system, wherein each heteroatom is independently selected from nitrogen, oxygen, phosphorous and sulfur ("5-18 membered heteroaryl").

As used herein, "heterocyclic," "heterocycloalkyl" or "heterocarbocyclic" each refer to any 3- to 18-membered non-aromatic radical monocyclic or polycyclic moiety comprising at least one heteroatom selected from nitrogen, oxygen, phosphorous and sulfur. A heterocyclic group can be a monocyclic, bicyclic, tricyclic or tetracyclic ring system, wherein the polycyclic ring systems can be a fused, bridged or spiro ring system. Heterocyclic polycyclic ring systems can include one or more heteroatoms in one or both rings. A heterocyclic group can be saturated or partially unsaturated. Partially unsaturated heterocycloalkyl groups can be termed "heterocycloalkenyl" if the heterocyclic contains at least one double bond, or "heterocycloalkynyl" if the heterocyclic contains at least one triple bond.

As used herein, the phrase "substituted" refers to a group in which one or more bonds to a hydrogen atom contained therein is replaced by a bond to a non-hydrogen or non-carbon atom. Examples of substituent groups include: halogens, hydroxyls, alkoxy, -CN, alkenyl, carboxyls, C₆-C₁₄ aryl, heterocyclic and so on.

As used herein, the term "acrylic compound" refers to acrylic acid or derivatives thereof with carboxylic acid group, including but not limited to acrylic acid, methacrylic acid, 2-carboxyethyl acrylate, itaconic acid, maleic acid and fumaric acid which may be used alone or in combination of two or more. The acrylic compound may undergo a ring-opening reaction with epoxide groups of the epoxidized vegetable oil so as to be grafted onto the epoxidized vegetable oil. Preferably, (meth)acrylic acid and/or 2-carboxyethyl acrylate are used. More preferably, acrylic acid and/or 2-carboxyethyl acrylate are used. Even more preferably, acrylic acid is used.

The symbol 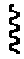 represents a point of attachment.

According to an embodiment of the present invention, based on 1 molar equivalent of the epoxy groups in the epoxidized vegetable oil, a total of molar equivalents of the Norrish Type II photoinitiator and the acrylic compound is 1 molar equivalent. The ratio of the molar equivalents of the Norrish Type II photoinitiator relative to the molar equivalents of the acrylic compound is preferably from 5:1 to 1:5, more preferably from 3:1 to 1:3.

According to an embodiment of the present invention, a polymerization inhibitor is further used in the ring-opening reaction. Suitable polymerization inhibitors may be selected from phenols such as hydroquinone, mono methyl ether of hydroquinone, mono-tert-butylhydroquinone, catechol, para-tert-butylcatechol, 2,6-di-tert-butyl-m-cresol, 2,6-di-tert-butyl-4-methylphenol, pyrogallol and β-naphthol; quinones such as benzoquinone, 2,5-diphenyl-p-benzoquinone, methyl-p-benzoquinone and p-xyloquinone; nitro compounds or nitroso compounds such as nitrobenzene, m-dinitrobenzene, 2-methyl-2-nitrosopropane, α-phenyl-tert-butyl nitrone and 5,5-dimethyl-1-pyrroline-1-oxide; amines such as tetrachlorobenzoquinone-amine, diphenylamine, diphenyl-picrylhydrazine, phenyl α-naphthylamine, pyridine and phenothiazine; and sulfides such as dithiobenzoyl sulfide and dibenzyl tetrasulfide. These polymerization inhibitors may be used alone or in a mixture of two or more.

According to an embodiment of the present invention, the photoinitiator of the present invention has a structural unit of formula (I): 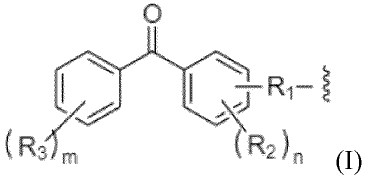 wherein
R₁ is selected from -COO-, -O-, -S-, -NR₄-, -CORs-O- or -OR₅-COO-;
R₂ and R₃ are independently selected from C₁₋₆ alkyl, C₁₋₆ alkoxy, hydroxyl, halogen, -COO-C₁₋₆ alkyl, C₆₋₁₀ aryl or -NR₄R₄; or R₂ and R₃ together form -S-, -O-, -CO- or -C=C-;
m is 0, 1, 2, 3 or 4;
n is 0, 1, 2 or 3;
R₄ is independently selected from H or C₁₋₆ alkyl; and
R₅ is selected from C₁₋₂₀ alkylene or C₂₋₂₀ alkenylene unsubstituted or substituted with one or more hydroxyl, halogen, C₁₋₆ alkoxy, or acyloxy groups. In case m is higher than 1, the R₃ groups can be part of a ring structure. In case n is higher than 1, the R₂ groups can be part of a ring structure. Preferably n and m are 0.

The present invention further provides a photoinitiator, which is prepared by a ring-opening reaction of epoxidized vegetable oil with one or more acrylic compounds and one or more compounds of formula (II), 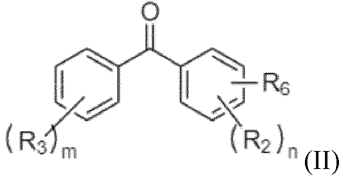 wherein R₂, R₃, R₆, m and n are as defined above.

According to an embodiment of the present invention, a polymerization inhibitor, such as mono methyl ether of hydroquinone, may be added to the ring-opening reaction.

The photoinitiator described above may be prepared by the reaction of the step shown in the following reaction scheme. The epoxidized vegetable oil is exemplified by the epoxidized soybean oil with the representative chemical formula shown on the left side of the reactants. The Norrish Type II photoinitiator is exemplified by the representative chemical formula shown on the upper right side of the reactants, and acrylic acid is further used as the reactant: 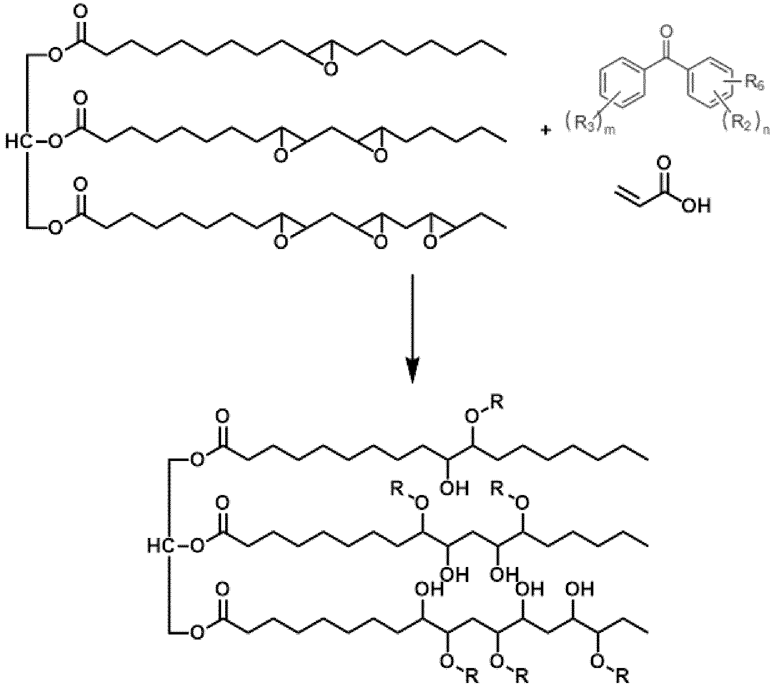 wherein R is the residue of the Norrish Type II photoinitiator or the acrylic acid after carrying out the ring-opening reaction with the epoxide group. In an embodiment of the present invention, the product epoxidized soybean oil photoinitiator of the above reaction scheme is prepared by the following steps: To a reaction vessel, 1 molar equivalent of epoxidized soybean oil, and a total of 6 molar equivalents of photoinitiator (such as benzophenone acid, BPA) and acrylic acid (in a ratio that may be arbitrarily adjusted, as long as the total is 6 molar equivalents) are added, and heated to 100°C under exclusion of light and under atmospheric pressure to react for 12 h, thereby obtaining the epoxidized soybean oil photoinitiator (ESBO-ABPA).

According to an embodiment of the present invention, in the method for preparing the photoinitiator, based on the total molar equivalents of the epoxidized vegetable oil, the total of molar equivalents of the Norrish type II photoinitiator and the acrylic acid is about the total molar equivalents of epoxide of the epoxidized vegetable oil. In an embodiment of the present invention, epoxidized soybean oil is used as the epoxidized vegetable oil raw material, and based on 1 molar equivalent of the epoxidized soybean oil, the total of molar equivalents of the Norrish type II photoinitiator and the acrylic acid is about 6 molar equivalents. The molar amount of the Norrish Type II photoinitiator relative to total molar amount of the Norrish Type II photoinitiator and of the acrylic compound preferably varies from 5 to 95%, more preferably from 30 to 70%, even more preferably from 40 to 60%.

As the exact composition of vegetable oils is variable (depends on factors such as soil conditions, climate and plant variety), the exact composition of an epoxidized vegetable oil is also variable. As a consequence, it is difficult to give an exact structure of the photoinitiators according to the invention. An idealized structure is depicted in formula (III).

Preferably, the photoinitiator is according to formula (III): 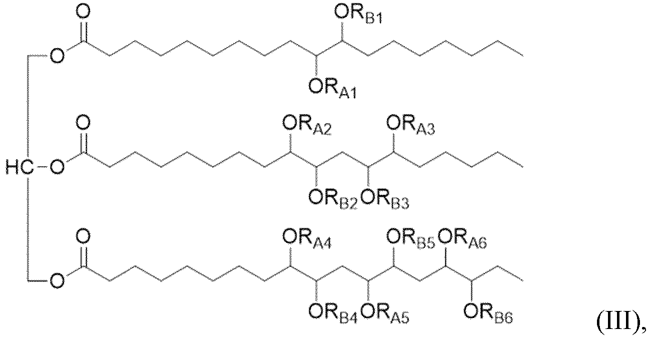 wherein R_{A1}, R_{B1}, R_{A2}, R_{B2}, R_{A3}, R_{B3}, R_{A4}, R_{B4}, R_{A5}, R_{B5}, R_{A6} and R_{B6} are independently selected from 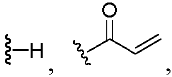 and 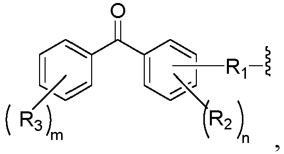 with the proviso that
(1) The total amount of 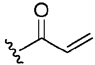 and 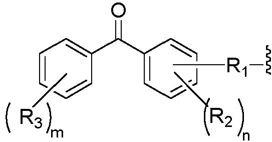 is from 4 to 6, preferably 5 or 6, more preferably 6;
(2) The total amount of 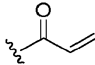 is from 1 to 5, preferably 2 or 3;
(3) The total amount of 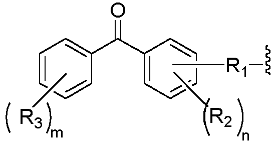 is from 1 to 5, preferably 2, 3 or 4, more preferably, 3 or 4;
(4) For each i, wherein i is from 1 to 6: if R_{Ai} is 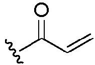 or 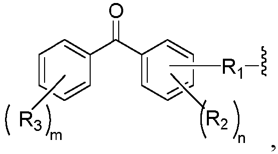 then the corresponding R_{Bi} is 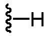 ; and
(5) For each i, wherein i is from 1 to 6: if R_{Bi} is 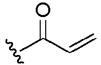 or 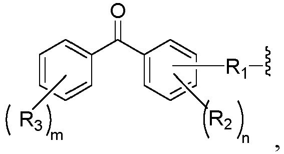 then the corresponding R_{Ai} is

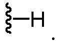

Accordingly, in this preferred embodiment of the invention, in case R_{A1}, R_{A2}, R_{A3}, R_{A4}, R_{A5}, or R_{A6}, respectively, is 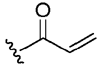 or 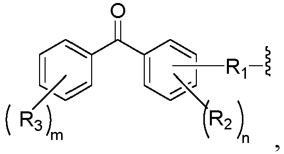 then R_{B1}, R_{B2}, R_{B3}, R_{B4}, R_{B5}, or R_{B6}, respectively is 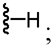 and in case R_{B1}, R_{B2}, R_{B3}, R_{B4}, R_{B5}, or R_{B6}, respectively, is 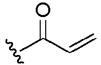 or 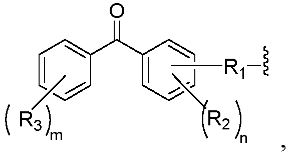 then R_{A1}, R_{A2}, R_{A3}, R_{A4}, R_{A5}, or R_{A6}, respectively is 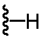 .

Undergo photopolymerization under short-wavelength light (200 nm to 400 nm), the photoinitiator of the present invention based on the epoxidized vegetable oil (e.g., ESBO-BPA) may cause UV curable resins (e.g. ethylenically unsaturated curable compounds) commonly used in industry to form cured coatings. These coatings may be subsequently applied in industry: for example, as optical fiber coatings, inks, varnishes or floor coatings, etc. In low-migration testing using 500 MHz Nuclear Magnetic Resonance (NMR), the inventors of the present invention surprisingly found that when the polymer photoinitiator (e.g., ESBO-BPA) of the present invention is used to cause photopolymerization of resin monomers, the polymer photoinitiator of the present invention has excellent low migration and high conversion efficiency. Due to the excellent low migration, the present invention can effectively reduce the possibility of harm to human health caused by the photoinitiator in the coating in industrial applications especially in indirect food contact applications.

The present invention further provides a photoinitiator according to formula (III): 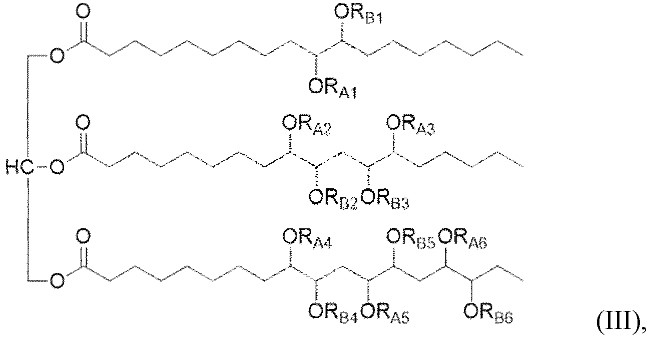 wherein R_{A1}, R_{B1}, R_{A2}, R_{B2}, R_{A3}, R_{B3}, R_{A4}, R_{B4}, R_{A5}, R_{B5}, R_{A6} and R_{B6} are independently selected from 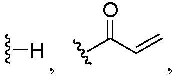 and 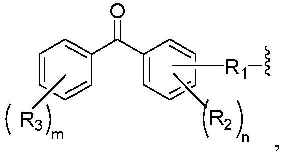 with the proviso that
(1) The total amount of 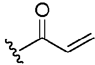 and 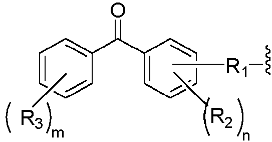 is from 4 to 6, preferably 5 or 6, more preferably 6;
(2) The total amount of 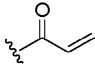 is from 1 to 5, preferably 2 or 3;
(3) The total amount of 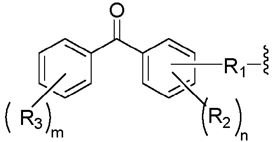 is from 1 to 5, preferably 2, 3 or 4, more preferably 3 or 4;
(4) For each i, wherein i is from 1 to 6: if R_{Ai} is 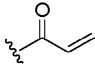 or 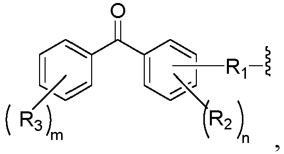 then the corresponding R_{Bi} is 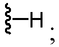 and
(5) For each i, wherein i is from 1 to 6: if R_{Bi} is 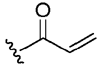 or 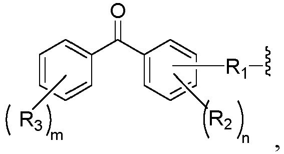 then the corresponding R_{Ai} is 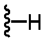 .

Accordingly, in case R_{A1}, R_{A2}, R_{A3}, R_{A4}, R_{A5}, or R_{A6}, respectively, is 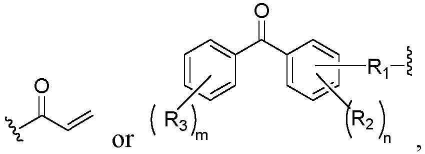 then R_{B1}, R_{B2}, R_{B3}, R_{B4}, R_{B5}, or R_{B6}, respectively is 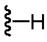 ; and in case R_{B1}, R_{B2}, R_{B3}, R_{B4}, R_{B5}, or R_{B6}, respectively, is 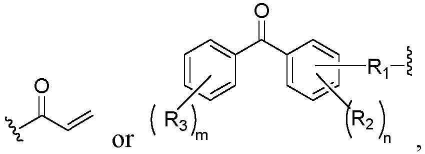 then R_{A1}, R_{A2}, R_{A3}, R_{A4}, R_{A5}, or R_{A6}, respectively is

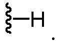

The present invention further provides a photocurable composition, including an ethylenically unsaturated curable compound and a photoinitiator as described above.

As used herein, the term "ethylenically unsaturated curable compound" means a compound containing polymerizable carbon-carbon double bonds. The polymerizable carbon-carbon double bond is a carbon-carbon double bond that can react with another carbon-carbon double bond in the polymerization. The polymerizable carbon-carbon double bond is usually selected from the group consisting of acrylate (including cyanoacrylate), methacrylate, acrylamide, methacrylamide, styrene, maleate, fumarate, itaconate, allyl, propenyl, vinyl and combinations thereof.

According to an embodiment of the present invention, the ethylenically unsaturated curable compound may be selected from (meth)acrylate functionalized monomers, (meth)acrylate functionalized oligomers, amine modified acrylates and mixtures thereof. As used herein, the term "(meth)acrylate functionalized monomers" means monomers containing a (meth)acrylate group, specifically an acrylate group. The term "(meth)acrylate functionalized oligomers" means oligomers containing a (meth)acrylate group, specifically an acrylate group. The term "(meth)acrylate group" includes an acrylate group (-O-CO-CH=CH₂) and a methacrylate group (-O-CO-C(CH₃)=CH₂).

Preferably, the photocurable composition comprises an oligomer and a reactive diluent. The oligomer is preferably an UV curable oligomer preferably selected from urethane (meth)acrylates, polyether urethane (meth)acrylates, polyester urethane (meth)acrylates, epoxy (meth)acrylates polyether acrylates and polyester (meth)acrylates.

For viscosity reasons it is in general beneficial that reactive diluents are used in the photocurable composition. With reactive diluent is meant a compound being able to reduce the viscosity of the formulation while being able to free radically copolymerize. As reactive diluent various acrylic, methacrylic or vinyl functional monomers can be used. Suitable examples are for instance diacrylates or dimethacrylates of diols or of polyetherdiols, such as propoxylated neopentyl glycol diacrylate, 1,6-hexanediol diacrylate, dipropylene glycol diacrylate (DPGDA), tripropylene glycol diacrylate (TPGDA), diethylene glycol diacrylate, triethylene glycol diacrylate, triethylene glycol dimethacrylate, neopentylglycol diacrylate, 1,4-butanediol diacrylate (e.g, SR213), alkoxylated aliphatic diacrylate (e.g. SR9209A), alkoxylated hexanediol diacrylate (e.g, SR561, SR562, SR563, SR564 from Sartomer Co., Inc), polyethylene glycol (200) diacrylate (SR259), polyether glycol-200-diacrylate, PEG300-diacrylate, polypropylene glycol diacrylate, ethoxylated (3) bisphenol-A-diacrylate, BDDA butanediol diacrylate, BDDMA butane diol dimethacrylate or higher functional acrylates such as trimethylolpropane triacrylate (TMPTA), ethoxylated trimethylolpropane triacrylate, TMP3EOTA ethoxylated (3) trimethylolpropane triacrylate, TMP6EOTA ethoxylated (6) trimethylolpropane triacrylate, propoxylated trimethylolpropane triacrylate, pentaerythritol triacrylate, pentaerythritol(4)-propoxylated triacrylate, pentaerythritol tetraacrylate, ethoxylated or propoxylated neopentylglycol, propoxylate (4) glycerol triacrylate, tri-functional monomers, such as Laromer types from BASF or Ebecryl 2047 or Ebecryl 12 from Allnex, ditrimethylolpropane tetraacrylate, dipentaerythritol-pentaacrylate (Di-PEPA), dipentaerythritol hexaacrylate (DPHA). Examples of vinyl compounds are compounds like butanediol divinyl ether or as mono functional compound N-vinyl caprolactam. Although mono functional (meth)acrylates like for example lauryl (meth)acrylate, phenoxyethyl (meth)acrylate can be used as well, it is preferred for low migratability to employ reactive diluents with at least two free radical curable ethylenically unsaturated groups. Obviously mixtures can be used as well. The reactive diluent is preferably selected from the group consisting of mono-, di-, tri-, tetra-, penta- and hexa-(meth)acrylates with a molecular weight lower than 800 g/mol and any combination of at least two thereof. The photocurable composition of the invention may comprise from 10 to 80 wt.%, in particular from 15 to 75 wt.%, more particularly from 20 to 70 wt.% (meth)acrylate-functionalized oligomer, relative to the entire weight of the photocurable composition. The one or more reactive diluents having one or more, preferably two or more, free radical curable ethylenically unsaturated groups may be present in the photocurable composition in an amount of at least 1 wt.%, or at least 5 wt.%, or at least 10 wt.%, and in an amount of at most 85 wt.%, or at most 80 wt.%, or at most 75 wt.%, or at most 70 wt.%, relative to the entire weight of the photocurable composition.

According to an example of the present invention, the photocurable composition of the present invention may further include an amine synergist. The amine synergist is introduced into the photocurable composition of the present invention in order to synergize with the photoinitiator and/or reduce oxygen inhibition. The amine synergist is typically a tertiary amine. When used in combination with the photoinitiator, the tertiary amine provides an active hydrogen donor site for exciting the triplet of the photoinitiator, thus generating reactive alkyl-amino radicals, which can subsequently initiate polymerization. The tertiary amine can also convert non-reactive peroxo species formed by the reaction between oxygen and radicals into reactive alkyl-amino radicals, thus reducing the influence of oxygen on curing.

The reaction mechanism of benzophenone and the amine synergist to generate radicals at 200 nm to 400 nm can be shown in the following reaction scheme:

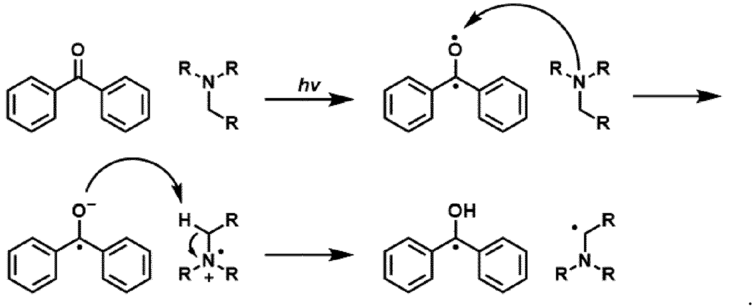

Examples of suitable amine synergists include low-molecular-weight tertiary amines (i.e., having a molecular weight of less than 200 g/mol), such as triethanolamine and N-methyldiethanolamine (MDEA). Other types of amine synergists are aminobenzoates. Examples of aminobenzoates include ethyl 4-(dimethylamino)benzoate (EDB), amyl 4-(dimethylamino)benzoate, 2-ethylhexyl 4-(dimethylamino)benzoate and 2-butoxyethyl 4-(dimethylamino)benzoate (BEDB). Next to these amine synergists, also acrylated amine synergists like AgiSyn^{™} 002, AgiSyn^{™} 003 and/or AgiSyn^{™} 008 can be suitably used. Next to these amine synergists, oligomeric amine synergists like AgiSyn^{™} 701 and/or AgiSyn^{™} 703 can be employed. An example of an oligomeric dimethylaminobenzoate is Omnipol ASA. The amine synergist can also be incorporated into the photoinitiator according to the invention. Especially when using acid functional amine synergists like dimethylamino benzoic acid, they can be readily incorporated during the synthesis of the photoinitiator. So according to an embodiment of the invention the photoinitiator comprises Norrish type II group(s), acrylic compound(s) and amine synergist group(s).

According to an embodiment of the present invention, in the photocurable composition, based on 1 molar equivalent of the total amount of the ethylenically unsaturated curable compound, the photoinitiator may be in the range of about 0.01 to about 0.1 molar equivalents (for example, about 0.05 molar equivalents); and the amine synergist may be in the range of about 0.03 to about 0.3 molar equivalents (for example, about 0.15 molar equivalents).

The examples disclosed herein are intended to illustrate the embodiments of the present invention and to explain the technical features of the present invention, and are not intended to limit the scope of the present invention. Any changes or equivalents that can be easily made by those skilled in the art are intended to be within the scope of the present invention. The scope of the present invention shall be subject to the scope of the appended claims.

### Comparative Experiment 1 Preparation of ESBO-BPA

1 g (1.025 mmol) of epoxidized soybean oil and 1.392 g (6.154 mmol) of 2-benzoylbenzoic acid were added to a 100 ml single-neck round-bottom flask, and heated to 100°C to react in the dark under atmospheric pressure for 12 h, thereby obtaining the epoxidized soybean oil photoinitiator (ESBO-BPA, which may include an exemplified component having a structure of the following chemical formula)

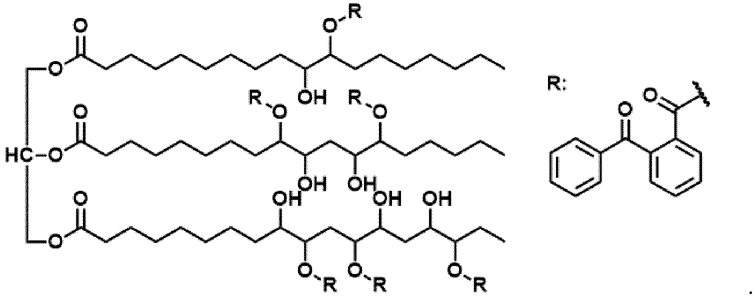

### Comparative experiment 2 preparation ofBP acrylate (BPHEA)

To a stirred mixture of 0.512g hydroxyethyl acrylate, 1.0g 2-benzoylbenzoic acid, and 0.054g 4-dimethylaminopyridine in CH₂Cl₂, a solution of 0.912g dicyclohexylcarbodiimide in CH₂Cl was slowly added at 0°C, under a nitrogen atmosphere. After the addition was complete, the temperature was raised to room temperature and the reaction mixture was stirred overnight. Next the reaction mixture was filtered and washed with 1 mol/L NaHCO₃ aqueous solution, saturated NaCl solution, and distilled water. After evaporation of the solvent the product was purified by silica gel column chromatography (ethyl acetate/hexane = 1:15) to obtain BPHEA.

### Example 1 Preparation of ESBO-ABPA

1 g (1.025 mmol) of epoxidized soybean oil, 0.696 g (3.078 mmol) of 2-benzoylbenzoic acid, 0.222 g (3.078 mmol) of acrylic acid, and 0.002 g of mono methyl ether of hydroquinone (MEHQ) as a polymerization inhibitor were added to a 100 ml single-neck round-bottom flask, and heated to 100°C to react in the dark under atmospheric pressure for 12 h, thereby obtaining the epoxidized soybean oil-acrylate photoinitiator (ESBO-ABPA, which may include an exemplified component having a structure of the following chemical formula)

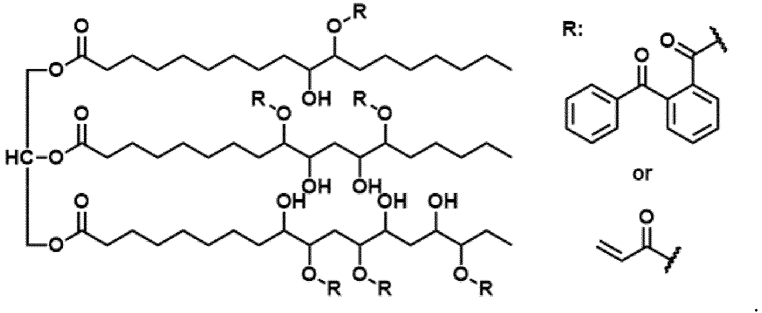

Structural identification by 500 MHz Nuclear Magnetic Resonance (NMR) showed that the epoxidized soybean oil photoinitiator (ESBO-BPA) and the epoxidized soybean oil-acrylate photoinitiator (ESBO-ABPA) had been successfully prepared.

The glass transition temperatures (Tg) of the polymer photoinitiators were measured by differential scanning calorimetry (DSC). Upon measurement, the glass transition temperatures of the epoxidized soybean oil photoinitiator (ESBO-BPA) and the epoxidized soybean oil-acrylate photoinitiator (ESBO-ABPA) were -1.84°C and -21.46°C, respectively.

### Example 2 Synthesis of Thin Film Polymer

1 molar equivalent of each of monomers tripropylene glycol diacrylate (TPGDA), 2-hydroxyethyl acrylate (HEA) and lauryl acrylate (LA) underwent a photopolymerization reaction, under short-wavelength light (365 nm) within 5 seconds, with 0.05 molar equivalents of photoinitiator (i.e., ESBO-BPA or ESBO-ABPA described above) and 0.15 molar equivalents of N-methyldiethanolamine as the amine synergist. Then, thin film polymers of these monomers can be obtained.

### Example 3 Conversion Rate Test

The monomer conversion rate was mearsured by using Fourier-transform infrared spectroscopy (FTIR). The conversion rate was determined as follows: tripropylene glycol diacrylate was taken as the monomer for testing, and its Fourier-transform infrared spectra before and after curing were obtained. The conversion rate could be calculated based on the ratio of carbonyl to propenyl. Upon measurement, the conversion rates of the epoxidized soybean oil photoinitiator (ESBO-BPA) and the epoxidized soybean oil-acrylate photoinitiator (ESBO-ABPA) were both 99.9%. The data shows that these photoinitiators can achieve high reactivity.

### Example 4 Migration Test

The obtained resin of tripropylene glycol diacrylate was used as the resin to be tested. The cured resin film was immersed in acetonitrile (ACN) and stirred at room temperature for 4 h. After the sample was filtered, its filtrate was taken, and mesitylene was added to the liquid to serve as an internal standard. The migration can be calculated from the integral ratio of mesitylene to photoinitiator according to the data of the nuclear magnetic resonance instrument. Upon measurement, the migration rates of the epoxidized soybean oil photoinitiator (ESBO-BPA) and the epoxidized soybean oil-acrylate photoinitiator (ESBO-ABPA) were respectively 8.7% and 0.8%, and the migration rate of the traditional benzophenone (BP) and BPHEA were 87% and 19%, respectively. The results are presented in Table 1.

**Table 1**

| | Photoinitiator | Migration rate (%) | Reduction factor in migration upon acrylation |
|---|---|---|---|
| Example 1 | ESBO-ABPA | 0.8 | 10.9 times |
| Comp 1 | ESBO-BPA | 8.7 | |
| Comp 2 | BPHEA | 19 | 4.6 times |
| Comp 3 | BP | 87 | |

The experimental results demonstrate a remarkably low migration level, as low as 0.8%, that can be achieved with the epoxidized soybean oil-acrylate photoinitiator of the present invention. This finding is particularly noteworthy when considering the comparative effects of acrylation of benzophenone: Acrylation of the epoxidized soybean oil (ESBO) photoinitiator results in a migration reduction factor of nearly 11-fold, despite the already low initial migration value of the ESBO photoinitiator. In contrast, acrylation of benzophenone photoinitiator yields a migration reduction factor of 4.6-fold (or nearly 5-fold). This substantial difference in migration reduction factors (11-fold vs. 5-fold) indicates an unexpected synergistic effect arising from the combination of ESBO and acrylation for obtaining low-migration photoinitiator.

## Claims

1. A photoinitiator, which is prepared by a ring-opening reaction of epoxidized vegetable oil with one or more Norrish Type II photoinitiators having carboxylic acid group, hydroxyl group, thiol group and/or amino group and one or more acrylic compounds.

2. The photoinitiator according to claim 1, wherein the epoxidized vegetable oil is epoxidized soybean oil.

3. The photoinitiator according to claim 1 or 2, wherein based on 1 molar equivalent of the epoxy groups in the epoxidized vegetable oil, a total of molar equivalents of the Norrish Type II photoinitiator and the acrylic compound is 1 molar equivalent.

4. The photoinitiator according to any one of claims 1 to 3, wherein the ratio of the molar equivalents of the Norrish Type II photoinitiator relative to the molar equivalents of the acrylic compound is from 5:1 to 1:5.

5. The photoinitiator according to any one of claims 1 to 4, wherein the photoinitiator has a structural unit of formula (I): 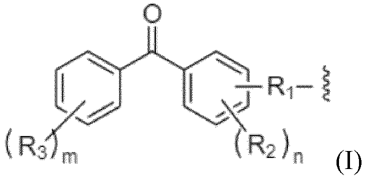 wherein
R₁ is selected from -COO-, -O-, -S-, -NR₄-, -CORs-O- or -OR₅-COO-;
R₂ and R₃ are independently selected from C₁₋₆ alkyl, C₁₋₆ alkoxy, hydroxyl, halogen, -COO-C₁₋₆ alkyl, C₆₋₁₀ aryl or -NR₄R₄; or R₂ and R₃ together form -S-, -O-, -CO- or -C=C-;
m is 0, 1, 2, 3 or 4;
n is 0, 1, 2 or 3;
R₄ is independently selected from H or C₁₋₆ alkyl; and
R₅ is selected from C₁₋₂₀ alkylene or C₂₋₂₀ alkenylene unsubstituted or substituted with one or more hydroxyl, halogen, C₁₋₆ alkoxy, or acyloxy groups,
with the proviso that in case m is higher than 1, the R₃ groups can be part of a ring structure and/or in case n is higher than 1, the R₂ groups can be part of a ring structure.

6. A photoinitiator, which is prepared by a ring-opening reaction of epoxidized vegetable oil with one or more acrylic compounds and one or more compounds of formula (II), 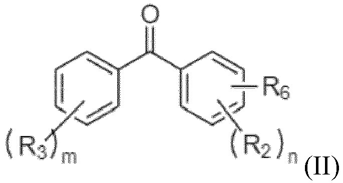 wherein
R₂ and R₃ are independently selected from C₁₋₆ alkyl, C₁₋₆ alkoxy, hydroxyl, halogen, -COO-C₁₋₆ alkyl, C₆₋₁₀ aryl or -NR₄R₄; or R₂ and R₃ together form -S-, -O-, -CO- or -C=C-;
m is 0, 1, 2, 3 or 4;
n is 0, 1, 2 or 3;
R₆ is selected from -OH, -SH, -NR₄R₄, -COOH, -CORsOH, or -OR₅COOH, wherein R₄ is independently selected from H or C₁₋₆ alkyl; and R₅ is selected from C₁₋₂₀ alkylene or C₂₋₂₀ alkenylene unsubstituted or substituted with one or more hydroxyl, halogen, C₁₋₆ alkoxy, or acyloxy groups,
with the proviso that in case m is higher than 1, the R₃ groups can be part of a ring structure and/or in case n is higher than 1, the R₂ groups can be part of a ring structure.

7. The photoinitiator according to claim 5 or 6, wherein m and n are 0.

8. The photoinitiator according to any one of the preceding claims, wherein a polymerization inhibitor is further added to the ring-opening reaction.

9. The photoinitiator according to any one of the preceding claims, wherein the one or more acrylic compounds are selected from the group consisting of methacrylic acid, acrylic acid and 2-carboxyethyl acrylate, preferably the acrylic compound is acrylic acid.

10. The photoinitiator according to any one of claims 1 to 9, wherein an amine synergist is incorporated into the photoinitiator.

11. The photoinitiator according to any one of the preceding claims, wherein the photoinitiator is according to formula (III): 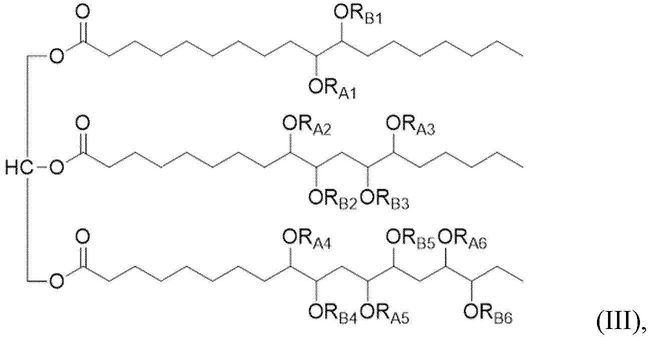 wherein R_{A1}, R_{B1}, R_{A2}, R_{B2}, R_{A3}, R_{B3}, R_{A4}, R_{B4}, R_{A5}, R_{B5}, R_{A6} and R_{B6} are independently selected from 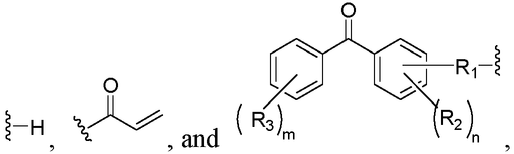 with the proviso that
(1) The total amount of 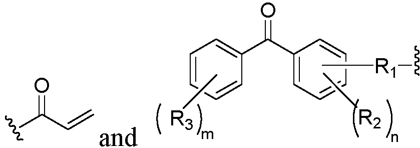 is from 4 to 6, preferably 5 or 6, more preferably 6;
(2) The total amount of 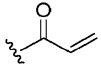 is from 1 to 5, preferably 2 or 3;
(3) The total amount of 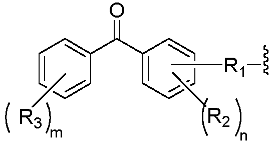 is from 1 to 5, preferably 2, 3 or 4, more preferably 3 or 4;
(4) For each i, wherein i is from 1 to 6: if R_{Ai} is 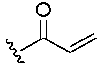 or 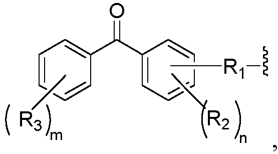 then the corresponding R_{Bi} is 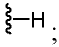 and
(5) For each i, wherein i is from 1 to 6: if R_{Bi} is 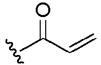 or 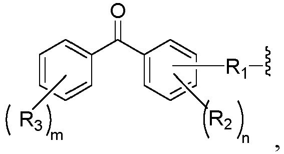 then the corresponding R_{Ai} is 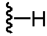 .

12. A photocurable composition, comprising an ethylenically unsaturated curable compound and the photoinitiator of any one of claims 1 to 11.

13. A photocurable composition of claim 12, wherein the photocurable composition comprises a (meth)acrylate functionalized oligomer and a (meth)acrylate functionalized monomer.
